# EUROPEAN PATENT APPLICATION

(11) **EP 2 586 466 A1**
(43) Date of publication of application: **01.05.2013**
(21) Application number: 11188811.1
(22) Date of filing: 11.11.2011
(51) Int. Cl.: A61L 9/20, C02F 1/32

(54) **Photocatalytic purification of media**

(30) Priority: 26.10.2011 US 551670 P
(71) Applicant: Philips Intellectual Property & Standards GmbH, 20099 Hamburg (DE); Koninklijke Philips Electronics N.V., 5621 BA Eindhoven (NL)
(72) Inventor: The designation of the inventor has not yet been filed
(74) Representative: Jungen, Carmen

(57) **Abstract**

The invention relates to the treatment of a medium, particularly to the purification of water, air, or surfaces. A photoactive layer (120) is disposed on an energy-transfer surface (111) of a substrate (110). Thus light energy transfer from said substrate (110) to the photoactive layer (120) is directly achieved without an intermediate passage through the medium. The substrate (110) may preferably be a waveguide from which light energy is transferred into the photoactive layer (120) via evanescent waves. Moreover, the optical coupling between the substrate (110) and the photoactive layer (120) may spatially vary.

## Description

### FIELD OF THE INVENTION

The invention relates to an apparatus and a method for the treatment of a medium, particularly for the purification of fluid media like water or air, or of surfaces.

### BACKGROUND OF THE INVENTION

US 2010/0209294 A1 discloses an apparatus for the photocatalytic purification of media like water or air. This apparatus comprises a container through which the medium to be treated is guided such that it surrounds a photocatalytic material. The photocatalytic material is activated by a light source, for example an LED that emits light into the medium via a waveguide.

### SUMMARY OF THE INVENTION

It is an object of the invention to provide means that allow for efficient photocatalytic treatment of media, particularly for the purification of water or air.

This object is achieved by an apparatus according to claim 1 and a method according to claim 2. Preferred embodiments are disclosed in the dependent claims.

According to its first aspect, the invention relates to an apparatus for the treatment of a medium, particularly for the purification of a fluid medium like water or air or of a solid medium like a surface. The apparatus comprises the following components:
a) A (firm or solid) substrate with a surface through which (light) energy is transferred during the operation of the apparatus. For the purpose of reference, said surface will in the following be called "energy-transfer surface".
b) A photoactive layer that is disposed (directly or indirectly) on the aforementioned energy-transfer surface and that can be contacted by the medium during the operation of the apparatus. In this context, the term "photoactive" shall refer to a material that can chemically react with substances in the medium under the influence of light, wherein said reaction may particularly comprise the enabling/initiating of reactions of other components.

According to a second aspect, the invention relates to a method for the treatment of a medium, particularly for the purification of water or air, said method comprising the following steps:
a) Absorption of light energy that is transmitted from a substrate, the absorption taking place in a photoactive layer that is disposed on an energy-transfer surface on said substrate.
b) Initiating (chemical) reactions in the medium by contacting it with the photoactive layer.

The apparatus and the method are based on the same inventive concept, i.e. the arrangement of a photoactive layer on an energy-transfer surface. Hence explanations and remarks provided for one of these realizations are valid for the other realization, too.

It is a central aspect of the apparatus and the method that the photoactive layer is optically coupled to the substrate in such a way that light from said substrate reaches the photoactive layer without prior passage through the medium. In this way losses of light within the medium can be avoided, leading to a more energy-efficient operation.

In the following, various preferred embodiments of the invention will be described that relate both to the apparatus and the method.

According to a first preferred embodiment, the photoactive layer comprises (or completely consists of) a photocatalytic material. As a catalyst, this material is not consumed during the operation but only mediates reactions of components of the medium, wherein the photocatalytic material is usually activated by the light received from the substrate. A typical example of a suitable photocatalytic material is TiO₂. Further examples may for example be found in the US 2010/0209294 A1.

The coupling or transfer of light energy from the energy-transfer surface to the photoactive layer preferably takes place by evanescent waves. Evanescent waves are for example generated when a light ray is totally internally reflected inside the substrate. An advantage of this approach is that energy transfer to the photoactive layer can be highly controlled and takes place only in the immediate vicinity of the energy-transfer surface, thus avoiding the loss of light energy into the medium.

The substrate may preferably comprise a waveguide, i.e. an element in which a (appropriately oriented) light beam propagates by repeated total internal reflections at the border of the waveguide. With the help of a waveguide, light can be transported and distributed to desired locations with minimal losses. Moreover, total internal reflections that take place in a waveguide lead to evanescent waves by which, as explained above, light energy can be coupled into the photoactive layer. Distribution and outcoupling of light can thus be achieved with minimal energy losses.

The energy that is transferred by the substrate into the photoactive layer may for example be ambient (solar) light that has previously been coupled into the substrate. According to a preferred embodiment, a technical light source is however provided for controllably generating light that is coupled into the substrate. The light source may for example comprise an LED or a laser diode, which allows generating light with a spectrum that can be tuned to the requirements of the photoactive layer. This also leads to improved energy efficiency.

There are different possibilities to couple light from the aforementioned light source into the substrate. An LED may for example be mounted immediately on a surface of the substrate. In a preferred embodiment, a reflecting element is provided between the light source and the substrate, allowing to dispose the light source at a convenient location while guaranteeing that its light reaches the substrate. The reflecting element may for example comprise a planar or no-planar (e.g. conical) mirroring surface, or a mirroring surface in the form of a Compound Parabolic Concentrator (CPC, cf. W.T. Welford, R. Winston, "High Collection Nonimaging Optics", Academic Press Inc (1990)). For optimal efficiency, if the substrate comprises a waveguide, the reflecting element should be designed such that the light from the light source enters the substrate under appropriate angles to achieve total internal reflection inside the waveguide.

According to a preferred realization of the invention, the above-mentioned light source is disposed in a cavity of the substrate. Surrounding the light source (as much as possible) by the substrate further minimizes losses of light energy.

The photoactive layer may immediately be deposited on the substrate. According to a preferred embodiment, an intermediate (solid) layer is deposited between the substrate and the photoactive layer, wherein total internal reflection takes place at the interface between this intermediate layer and the substrate during operation. The intermediate layer is usually characterized by a lower refractive index than the substrate, i.e. it acts as a "cladding layer". The substrate can hence be designed as a waveguide as explained above. Providing an intermediate layer between such a waveguide and the photoactive layer has the advantage that waveguide properties and light outcoupling properties can separately be adjusted and are particularly independent of the optical properties of the photoactive layer. More particularly, the evanescent waves generated during total internal reflection at the interface between substrate and intermediate layer are largely confined to the intermediate layer and might penetrate into layers in contact with the intermediate layer. Only the part of the evanescent wave extending outside the intermediate layer, i.e. into adjacent layers, can be used for energy transfer. The photocatalytic material can thus be partially decoupled from the substrate (waveguide) by tuning the refractive index and thickness of an intermediate (cladding) layer. Such a decoupling allows to distribute illumination across a large area, also into regions remote from the light source. If, on the contrary, the photoactive layer is directly on top of a waveguide, direct absorption of light passing through the layer will occur instead (in proportion to the absorption coefficient and the path length in this layer) due to partial absorption of the evanescent wave leaking out of the waveguide. This direct absorption of the refracted wave will be relatively strong, limiting the illumination to the close vicinity of the light source. A further advantage of a decoupling via an intermediate (cladding) layer is that it enables the use of scattering photocatalytic coatings (e.g. of a photocatalytic layer consisting of particles, ranging from less than a complete layer to a thick layer). With direct application of a photocatalyst on a waveguide always a non-scattering film should be used, as in that case scattering directly breaks down the waveguide functionality.

It should be noted that, instead of considering the aforementioned intermediate layer as a new component of its own, it might as well formally be considered as a part of the light emitting layer or of the photoactive layer. Moreover, if the photoactive layer has appropriate optical properties (e.g. a lower refractive index than the substrate, non-scattering structure), it might itself act as a "cladding layer" on the substrate.

The design and the optical characteristics may be homogeneous across the energy-transfer surface of the substrate. In a preferred embodiment of the invention, the design is however such that the optical coupling between the substrate and the photoactive layer varies spatially across the energy-transfer surface. In this way the intensity with which light is coupled into the photoactive layer can spatially be adjusted according to the needs of the application at hand. A homogeneous supply of light energy to the photoactive layer can for example be achieved by reducing the optical coupling at locations of high light intensity in the substrate (e.g. close to a light source).

The substrate, the photoactive layer, and/or the intermediate layer may have a thickness that spatially varies across the plane of the energy-transfer surface. By such thickness variations, optical characteristics can be adjusted as desired. The aforementioned varying optical coupling between the substrate and the photoactive layer can for example be achieved by varying the thickness of an intermediate layer appropriately.

According to another embodiment of the invention, the substrate is adapted to generate by itself the light that it transfers into the photoactive layer. In this case no external light source and no means to transfer light from such a source into the substrate are necessary.

The substrate may particularly comprise (or be) an Organic Light Emitting Diode (OLED). This realizes an embodiment of the aforementioned design. OLEDₛ have the advantage that they can be produced with flexible shapes and large surface areas. Accordingly, no light distribution layer is necessary as light is generated immediately at the location from where it is transferred into the photoactive layer.

The invention further relates to the use of the described apparatus for a photoreactive treatment of a medium, particularly for a photocatalytic treatment. This treatment may preferably comprise the purification of water, air and/or surfaces, for example
- in drinking water purification devices, UV water disinfection lamps, swimming pools, green houses, aquaria, aqua culture, industrial processes employing water,
- in air conditioners, fume hoods, luminaires,
- on door handles or other surfaces used by many people in public spaces.

### BRIEF DESCRIPTION OF THE DRAWINGS

These and other aspects of the invention will be apparent from and elucidated with reference to the embodiments described hereinafter.

In the drawings:
- Fig. 1: schematically shows a side view of an apparatus according to the present invention;
- Fig. 2: illustrates the optical situation at the interface between the substrate and the photoactive layer;
- Fig. 3: shows a CDC between the light source and the light emitting layer;
- Fig. 4: shows an LED die next to the substrate;
- Fig. 5: shows the arrangement of an LED in a cavity of the substrate;
- Fig. 6: is a diagram showing the absorbance versus the internal angle for refractive indices of the intermediate layer ranging from 2.25 to 1.40, while the substrate has a refractive index of 1.45.

Like reference numbers refer in the Figures to identical or similar components.

### DETAILED DESCRIPTION OF EMBODIMENTS

Photocatalytic oxidation (PCO) is a method for initiating reactions with light and can also be used as method for (partial) cleaning of water, air, or surfaces. PCO is based on the activation of the surface of a photocatalyst by incident light. Upon light absorption active surface states are generated. These active states are subsequently able to react with species adsorbing on or impinging at the surface. In this way organic compounds can effectively be decomposed. The photocatalyst can be applied in several ways. Examples are as a monolithic material or coated onto a supporting surface, either as a thin film or as a particulate coating, which comes into contact with the medium (water, air and other solvents) to be purified. A typical photocatalyst is TiO₂ in contact with water or air. For excitation of TiO₂ radiation with a wavelength shorter than 400 nm is typically required, though specific modifications thereof can also be used with visible light (cf. J. Tao, T. Luttrell, M. Batzill: "A two-dimensional phase of TiO2 with a reduced bandgap", NATURE CHEMISTRY, Vol. 3, April 2011, published at www.nature.com/naturechemistry). Materials activated by irradiation with blue light that can be used comprise e.g. TiONₓ.

In US 2010/0209294 A1, excitation of a photocatalysts is achieved with the help of a direct light source. The use of a direct light source has however several disadvantages. First, the light in many cases already has to pass the medium to be cleaned. If this is e.g. turbid water, only a limited fraction of the light flux actually reaches the active surface. Second, as the incident angle of the light at the active surface is not controlled, a large fraction of the light will pass the active material without being absorbed unless very thick layers of the active material are used.

In view of this, an air/water/surface purification/disinfection device is proposed consisting of a (e.g. planar or tubular) waveguide to transfer energy to the active surface. The photocatalyst is coated onto a cladding layer on the waveguide surface.

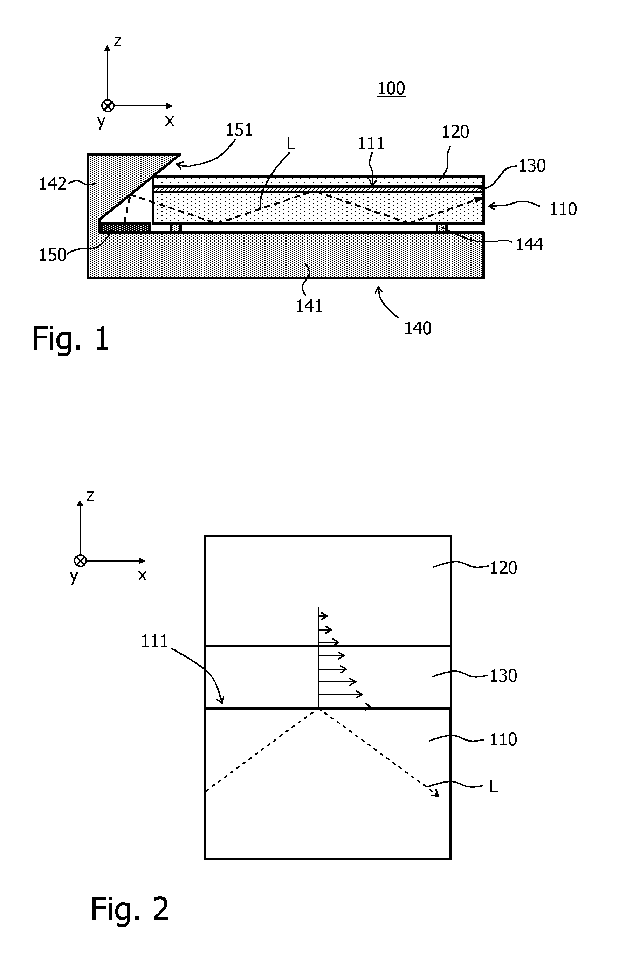

Figure 1 schematically shows a side view of an apparatus 100 that is designed according to the aforementioned principle. As a central component, the apparatus 100 comprises a substrate 110, here realized by a planar waveguide extending in x- and y-direction. The upper surface of this waveguide 110 constitutes an energy-transfer surface 111 on which a photoactive layer 120, here a photocatalyst like TiO₂, is disposed. In the shown embodiment, the photocatalyst 120 is not directly disposed on the waveguide 110, but indirectly via an intermediate layer 130.

The described components are arranged in a housing or support 140. The housing 140 comprises a support plate 141, e.g. of aluminum, which also acts like a cooling element for the heat dissipation generated by the (preferably UV or blue) LED(s) 150 that are also disposed on it. The light incoupling in the waveguide 110 is attained by an aluminum (or a high reflective non UV or blue absorbing material or stack of materials) wedge 142 above the LEDs 150. The waveguide 110 is supported by the aluminum plate 141 by four points 144 of support. This way the waveguide can by aligned with the height of the LED. The points can be of any material as they only occupy a small part of the surface, but preferably they are optically decoupled from the waveguide by having, at least at the interface with the waveguide, a refractive index lower than that of the waveguide. The waveguide material may be quartz glass or another material which does not or hardly absorb at the wavelength of the transported light.

With the described approach, the irradiation of the photocatalyst 120 is most efficient as the waveguide 110 can be operated such that the light L travels in total internal reflection. This implies that light that is not absorbed in a single pass of the active layer 120 does not leave the waveguide 110, but might be absorbed in a subsequent pass. This also implies that very thin layers of the active photocatalytic material may be used. Also, when the active material is discontinuously covering the surface 111 (e.g in the case of patterned films or by applying a particle coating), this does not lead to light losses as a consequence of light leaving the device without passing through the photocatalytic layer. Losses originating from the medium are not present, as the light does not travel through the (to be purified) medium, which often can be hazy.

An additional advantage of the described approach is that the light distribution, i.e. the local power density available for PCO, can be tuned in several ways. Most straight forward, but strongly limited, is a tuning by appropriate dimensioning of waveguide thickness (i.e. extension in z-direction) and thickness of the photocatalyst 120. A more versatile method of tuning is by using an intermediate or cladding layer 130 with a refractive index lower than that of the waveguide 110. Such an intermediate layer 130 decouples the total internal reflection in the waveguide 110 from refraction into the TiO₂ layer 120. By properly tuning thickness and refractive index of this intermediate layer 130, the fraction of light that can couple into the photocatalyst 120 can be tuned (related to overlap with the evanescent wave). Typical values for the thickness of the intermediate layer 130 range between about 50 nanometer and about 5 micrometer. The thickness of the photoactive layer 120 is not critical and may range from a few 10s of nanometers to many micrometers. The local power density available for PCO might further be tuned by locally varying the thickness of the photocatalytic layer on top of the intermediate layer or by varying the pattern density in the photocatalytic layer.

The described design benefits from using low etendue light sources like LED_{S} and laser diodes, as these will enable high coupling efficiency from light source to waveguide and also allow for exact tuning of the emitted wavelength to the excitation energy of the photocatalyst.

Figure 2 comprises an illustration of the basic way in which the light distribution is achieved by making use of total internal reflection (TIR). Total internal reflection of a light beam L is attained by making use of an intermediate layer 130 (or "cladding layer") with a low refractive index n₁ smaller than the refractive index no of the waveguide substrate 110. Due to the limited overlap of the evanescent wave (indicated by parallel arrows) with the photocatalyst 120, only a small fraction of the light is absorbed by the photocatalyst. When light reflects at the upper waveguide surface, the evanescent wave penetrates the cladding layer 130 and a small portion is absorbed in the photocatalyst 120. This portion decreases with increasing refractive index difference between waveguide and cladding and with increasing cladding layer thickness.

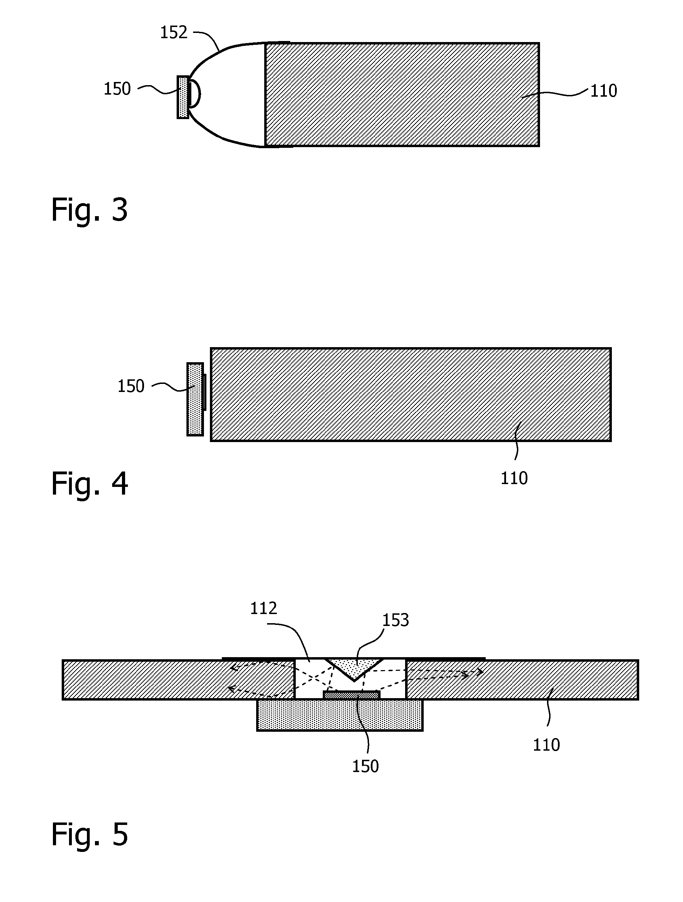

There are several ways how light can be coupled from a light source 150 into the waveguide 110. All these ways are suitable. Here a few examples are given. In Figure 3, an LED 150 is placed a small distance away from the waveguide 110, and mirrors 152 in the form of a Compound Parabolic Concentrator (CPC) are placed at both sides to reflect the light from larger angles into the waveguide.

Figure 4 shows another embodiment, in which the LED die 150 is placed directly at the side of the waveguide 110 (with a small air gap or in contact).

As illustrated in Figure 5, light can also be coupled into the waveguide 110 by placing an LED 150 in a hole or cavity 112 of the waveguide 110 with a (e.g. conical) reflector 152 on top. This way a very thin waveguide can be used (thickness about 1 mm).

Using laser diodes, waveguides with a thickness of a few tens of micrometers are possible as the spot size is small and the incoupling angle can be easily controlled.

The described approach can be varied in many ways. The waveguide may for example be provided with grooves of different shapes and with different distances between them (not shown) to adjust a desired light distribution in the waveguide. In addition, the thickness of the waveguide may be non-uniform.

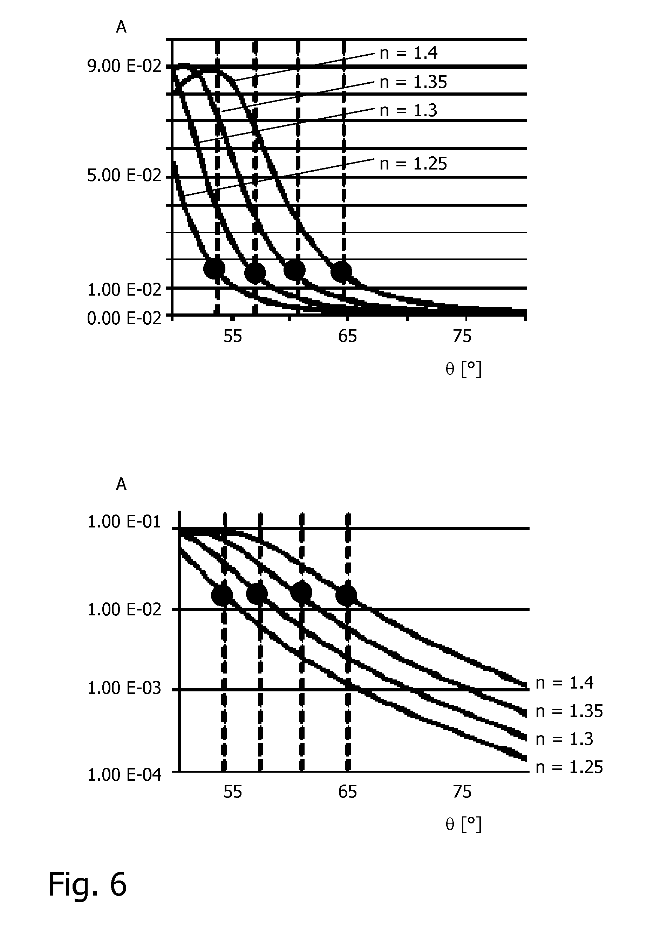

Figure 6 depicts two diagrams plotting the absorbance A = log (I₀/I) in the photocatalyst as a function of the internal angle θ for different refractive indices n1 (ranging from 1.25 to 1.4 with a waveguide with index 1.45) of the intermediate cladding layer 130. The thickness of the cladding layer 130 is assumed as 200 nm and the thickness of the photocatalyst (TiO₂) as 100 nm. Dashed vertical lines indicate the critical angles (light transport through the waveguide takes place at angles exceeding the critical angle).

The described apparatus and procedure can for example be applied for air purification in air conditioners, kitchen fume hoods, lab fume hoods, luminaires in store rooms (e.g. for chemicals, solvents, fuels etc.), luminaires in toilets, luminaires in gymnasia, luminaires in hospitals, and for water purification in mobile drinking water purification devices, stationary drinking water purification devices, swimming pools (reducing risk of infection by e.g. legionellae), green houses, aquaria, aqua culture, industrial processes employing water etc. Moreover, it can be applied for surface purification, e.g. on door handles in public spaces, or for surface cleaning, e.g. of luminaires (especially when maintenance costs are high).

The invention can also be applied in UV water disinfection lamps that are permanently in contact with water. Biofilm formation on the UV exit window of the luminaire would severely reduce the UV transmission of the exit window. The invention can be used to prevent biofilm formation on the exit window. Also in other systems where biofilm formation hampers functionality, the invention can be applied.

In summary, the invention relates to an air/water/surface purification/disinfection device comprising a waveguide to illuminate the active surface. The photocatalyst is coated onto the waveguide surface. A crucial aspect is the way in which outcoupling of light can be achieved, i.e. by tuning the intensity of an evanescent wave. This means that the (local) intensity of the light escaping via the (photocatalytically active) surface is determined by the refractive index and thickness of a kind of decoupling layer (intermediate layer) in between the waveguide in which the light propagates and the photoactive layer in which the photocatalytic oxidation takes place. The main advantages of the invention are:
- A strongly decreased light loss as the light is trapped inside the waveguide by total internal reflection and can only be adsorbed in the active material, and is hardly transmitted into medium (in contrast to conventional direct light sources).
- The light transportation mechanism allows the use of very thin films of photocatalyst without introducing transmission losses.
- The light does not have to pass the medium to be purified (which often leads to loss of light intensity).
- The light distribution can be controlled over a large surface area by using a low-index intermediate layer (evanescent wave coupling).
- The use of low etendue light sources like LED_{S} and laser diodes, which enables high coupling efficiency from light source to waveguide and also allows for exact tuning of the emitted wavelength to the excitation energy of the photocatalyst, again improving energy efficiency.
- The use of light sources like LED_{S} that also allows the use of light algorithms i.e. using optimized pulse schemes. Such schemes might in addition interact with a sensor that detects e.g. the level of contamination.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An apparatus (100) for the treatment of a medium, particularly for the purification of fluid media like water or air or of surfaces, comprising:
a) a substrate (110) with an energy-transfer surface (111) through which energy is transferred during operation of the apparatus (100);
b) a photoactive layer (120) that is disposed on the energy-transfer surface (111) and that can be contacted by the medium.

2. A method for the treatment of a medium, particularly for the purification of fluid media like water or air or of surfaces, comprising the following steps:
a) transfer of light energy from a substrate (110) into a photoactive layer (120) that is disposed on an energy-transfer surface (111) of the substrate (110);
b) initiating reactions in the medium by contacting it with the photoactive layer (120).

3. The apparatus (100) according to claim 1 or the method according to claim 2,
**characterized in that** the photoactive layer (120) comprises a photocatalytic material.

4. The apparatus (100) according to claim 1 or the method according to claim 2,
**characterized in that** light energy is transferred from the substrate (110) to the photoactive layer (120) by evanescent waves.

5. The apparatus (100) according to claim 1 or the method according to claim 2,
**characterized in that** the substrate (110) comprises a waveguide.

6. The apparatus (100) according to claim 1 or the method according to claim 2,
**characterized in that** a light source (150) is provided for generating light that is coupled into the substrate (110).

7. The apparatus (100) or the method according to claim 6,
**characterized in that** the light source (150) comprises an LED and/or a laser diode.

8. The apparatus (100) or the method according to claim 6,
**characterized in that** a reflecting element (151, 152, 153) is provided between the light source (150) and the substrate (110).

9. The apparatus (100) or the method according to claim 6,
**characterized in that** the light source (150) is disposed in a cavity (112) of the substrate (110).

10. The apparatus (100) according to claim 1 or the method according to claim 2,
**characterized in that** an intermediate layer (130) is disposed between the substrate (110) and the photoactive layer (120), wherein total internal reflection takes place at the interface between said intermediate layer and the substrate during operation.

11. The apparatus (100) according to claim 1 or the method according to claim 2,
**characterized in that** the optical coupling between the substrate (110) and the photoactive layer (120) varies spatially across the energy-transfer surface (111).

12. The apparatus (100) according to claim 1 or the method according to claim 2,
**characterized in that** the substrate (110), the photoactive layer (120), and/or the intermediate layer (130) have a spatially varying thickness.

13. The apparatus (100) according to claim 1 or the method according to claim 2,
**characterized in that** the substrate (110) generates the light that is emitted into the photoactive layer (120).

14. The apparatus (100) according to claim 1 or the method according to claim 2,
**characterized in that** the substrate (110) comprises an OLED.

15. Use of the apparatus (100) according to claim 1 or 3-14 for the photoreactive treatment of a medium, particularly for the purification of water, air and/or surfaces, for example
- in drinking water purification devices, UV water disinfection lamps, swimming pools, green houses, aquaria, aqua culture, industrial processes employing water,
- in air conditioners, fume hoods, luminaires,
- on door handles in public spaces.
